# EUROPÄISCHE PATENTSCHRIFT

(11) **EP 2 927 681 B1**
(45) Veröffentlichungstag und Bekanntmachung des Hinweises auf die Patenterteilung: **13.02.2019**
(21) Anmeldenummer: 15161657.0
(22) Anmeldetag: 30.03.2015
(51) Int. Cl.: G01N 30/06, B01L 3/00, G01N 30/60, G01N 30/88, G01N 33/28, G01N 30/14

(54) **VORRICHTUNG ZUR EXTRAKTION UND ANALYSE VON GASEN**
DEVICE FOR THE EXTRACTION AND ANALYSIS OF GASES
DISPOSITIF D'EXTRACTION ET D'ANALYSE DE GAZ

(30) Priorität: 31.03.2014 CH 4912014
(43) Veröffentlichungstag der Anmeldung: 07.10.2015
(73) Patentinhaber: Inrag AG, 4127 Birsfelden (CH)
(72) Erfinder: Adlhart, Christian, 8800 Thalwil (CH); Schneider, Frank, 79618 Rheinfelden (DE); Hug, Thomas Silvan, 8703 Erlenbach (CH)
(74) Vertreter: Bohest AG

(56) Entgegenhaltungen:
- WO-A1-01/95990
- WO-A1-2009/024171
- DE-B3-102010 008 066
- FR-A1- 2 988 620
- US-A1- 2009 308 246
- RAM AWATAR MAURYA ET AL: "Continuous In Situ Generation, Separation, and Reaction of Diazomethane in a Dual-Channel Microreactor", ANGEWANDTE CHEMIE INTERNATIONAL EDITION, Bd. 50, Nr. 26, 25. Mai 2011 (2011-05-25), Seiten 5952-5955, XP055204635, ISSN: 1433-7851, DOI: 10.1002/anie.201101977

## Beschreibung

Die Erfindung betrifft das Gebiet der Gasextraktion und dessen Analyse, insbesondere der Gasextraktion und Analyse von Transformatorengasen.

Es ist bekannt, dass wenn Transformatorengase nicht überwacht und wiederaufbereitet werden, diese hochexplosive Gasgemische bilden können. Deshalb werden Transformatoren regelmässig Ölproben entnommen und analysiert. Zur Gasanalyse werden vorzugsweise Gaschromatographen verwendet. Kontinuierliche Überwachungen vor Ort sind wünschenswert, da sie zur Sicherheit beitragen können. Online-Messgeräte mit herkömmlichen Gaschromatographen sind jedoch gross und teuer in Anschaffung und Unterhalt.

In WO 01/95990 und US 2009/0308246 wird ein Gasextraktionsmodul mit einem herkömmlichen Gaschromatographen verbunden offenbart.

Es besteht somit Bedarf an Vorrichtungen, welche eine kontinuierliche Gasextraktion und Gasanalyse vor Ort erlauben und möglichst kostengünstig sind.

Basis der Erfindung ist es, ein in Mikro-Technik implementiertes Gaschromatographen-Modul mit einem ebenfalls miniaturisierten Gasextraktionsmodul zu kombinieren, vorzugsweise mit möglichst geringen Totvolumina. Mit der Vorrichtung soll eine kontinuierliche Analyse und Überwachung eines Gasgehalts in einer Flüssigkeit, insbesondere des Ölgasgehalts bei Leistungstransformatoren ermöglicht werden.

Nach einem ersten Aspekt der Erfindung wird eine Vorrichtung zum Extrahieren und Analysieren von Gas nach Anspruch 1 bereitgestellt. Die Vorrichtung beinhaltet einen Gaschromatograph mit einem miniaturisierten Chromatographen-Modul, welches auf einer Trägerplatte angeordnet einen Injektor, eine mit einer Heizung verbundene Trennsäule, einen Detektor, sowie mehrere Kapillaren zum Verbinden dieser Funktionsteile zur Zu- und Abfuhr von Trägergas und Probengas aufweist. Die Vorrichtung beinhaltet zudem ein miniaturisiertes Gasextraktionsmodul mit mindestens einem Gasextraktor mit einer gaspermeable Membran zum Extrahieren von Gas aus einer Flüssigkeit durch die Membran. Dabei bildet die Membran mindestens eine Längswand eines Kanals im Gasextraktor. Beispielsweise können ein oder mehrere Kanäle im Gasextraktor durch eine oder mehrere Membrane gebildet werden. Das Gasextraktionsmodul weist mehrere Anschlussleitungen zum Verbinden des Gasextraktionsmoduls mit einer Flüssigkeitszufuhr und -abfuhr, sowie mit einer Extraktionsgaszufuhr und mit einer Extraktions- und Probengasabfuhr auf. Typischerweise ist eine Extraktions- und Probengasabfuhr in einer Leitung kombiniert. Eine Probengasabfuhrleitung des Gasextraktionsmoduls ist mit einer Kapillare zur Zufuhr von Probengas verbunden und bildet einen Verbindungsabschnitt einer Probengasleitung. In der Vorrichtung gemäss vorliegender Erfindung ist der Verbindungsabschnitt der Probengasleitung zwischen Gasextraktionsmodul und Injektor des Chromatographen-Moduls ventilfrei. Beinhaltet ein Gasextraktionsmodul mehrere Gasextraktoren und mehrere entsprechende Verbindungsabschnitte der Probengasleitung zwischen dem Gasextraktionsmodul und dem oder gegebenenfalls auch den mehreren Injektoren des Chromatographen-Moduls, dann sind vorzugsweise sämtliche dieser Verbindungsabschnitte der Probengasleitung ventilfrei.

Die Kombination eines miniaturisierten Gaschromatographen und eines miniaturisierten Gasextraktionsmoduls bietet zum einen die Vorteile der Miniaturisierung der einzelnen Komponenten. Dies sind beispielsweise ein geringer Gasverbrauch (Trägergas, Extraktionsgas, Kalibrationsgas), was eine lange Betriebsdauer der Vorrichtung ohne Unterhalt erlaubt. Dies wiederum senkt die Unterhaltskosten der Vorrichtung. Zudem können bereits kleine Probengasmengen analysiert werden. Auch sind Messzeiten in der Regel sehr kurz (Messzyklen von wenigen bis wenigen zehn Minuten) . Auch erzeugt ein Aufheizen einer kleinen Trennsäule nur geringe Abwärme, was zu einem besseren Gesamtenergiehaushalt beiträgt. Der durch die miniaturisierten Komponenten ermöglichte kompakte Aufbau einer Vorrichtung erlaubt zudem das Herstellen einer portablen Ausführung der Vorrichtung, sowie allgemein eine leichte Handhabung und Installation der Vorrichtung. Der kompakte Aufbau ist insbesondere bei beengten Platzverhältnissen von Vorteil. Dies kommt vor allem dann zum Tragen, wenn eine Vorrichtung permanent in eine, eventuell bereits bestehende Installation integriert werden soll, wie dies für kontinuierliche, online Analysen erforderlich ist.

Die erfindungsgemässe Kombination von Gaschromatograph und Gasextraktionsmodul bietet jedoch weitere Vorteile. Ein ventilfreier Verbindungsabschnitt der Probengasleitung zwischen dem Gasextraktionsmodul und dem Chromatographen-Modul des Gaschromatographen vereinfacht und vergünstigt nicht nur den Aufbau der Vorrichtung aufgrund weniger Bestandteile, die verbaut und gewartet werden müssen. Ein ventilfreier Verbindungsabschnitt der Probengasleitung kann bis auf ein Minimum verkürzt und damit ein Totvolumen der Vorrichtung und eine Ansprechzeit der Vorrichtung minimal gehalten werden. Insbesondere ermöglicht die erfindungsgemässe Vorrichtung auch das integrale Ausbilden von Ab- und Zufuhrleitungen und ebensolchen Kapillaren von Injektor und Gasextraktor, und des dazwischen angeordneten Verbindungsabschnitts der Probengasleitung. Je nach Ausgestaltung eines Gasextraktors ist damit die Fertigung von Gaschromatographen-Modul und Gasextraktor als integrales Bauteil möglich. Der ventilfreie Verbindungsabschnitt bildet ein zusammenhängendes Volumen, das beispielsweise als durchgehende Leitung mit verschiedenen Durchmessern realisierbar ist. Dabei können einzelne Leitungsabschnitte fest oder lösbar miteinander verbunden sein, beispielsweise integral geformt, miteinander verschraubt, geklemmt oder verschweisst sein.

Generell ist eine Fluidik in der erfindungsgemässen Vorrichtung sehr einfach gehalten. In einer sehr einfachen Basisversion der Vorrichtung werden lediglich zwei Präzisionsventile, sowie ein Druckreduzierventil für eine Trägergas- oder Extraktionsqasquelle benötigt. Wird die Vorrichtung mit einer Kalibrationsgasquelle ergänzt, kann dies über ein zusätzliches Druckreduzierventil erfolgen, falls die Präzisionsventile entsprechend als Mehrwegventile (Zwei- bis Drei-Wegventil) ausgebildet sind. Die kompakte Bauweise und insbesondere die kurzen Probengaswege erlauben das Ausnutzen der Diffusion des Probengases entlang des Verbindungsabschnitts der Probengasleitung bis in einen Injektionsloop im Gaschromatographen-Modul bereits während eines Messzyklus. Aufgrund der minimal gehaltenen Wege für das Probengas zwischen Gasextraktionsmodul und Chromatographen-Modul, kombiniert mit dem bereits minimalen Weg der Kapillaren bis zum Injektor im Chromatographen-Modul, wird ein Injektionsloop im wesentlichen Teil des Extraktionsmoduls. Die Module für Extraktion und Analyse sind in der erfindungsgemässen Vorrichtung derart miteinander kombiniert, dass eine Gasführung im Vergleich zu herkömmlichen Gasführung zwischen zwei kombinierten Modulen vereinfacht und verkürzt wird. Insbesondere sind keine Ventile in einer Probengasleitung insbesondere im Verbindungsabschnitt der Probengasleitung erforderlich. Zudem ist ein Analysemodul bereits in einen Prozessablauf involviert, wenn eine Gasextraktion im Extraktionsmodul noch aktiv ist.

Miniaturisierte Chromatographen-Module (GC-Module) wie sie in der erfindungsgemässen Vorrichtung vorzugsweise Verwendung finden sind bekannt und sind beispielsweise im Dokument EP 1 588 156 im Detail beschrieben. Es wird deshalb darauf verzichtet ein miniaturisiertes GC-Modul im Detail zu beschreiben und es sei dazu auf das Dokument EP 1 588 156 verwiesen. Der prinzipielle Aufbau eines Gaschromatographen mit miniaturisiertem Chromatographen-Modul wie es in der erfindungsgemässen Vorrichtung vorzugsweise verwendet wird, ist zudem beispielsweise in der europäischen Patentanmeldung EP 2 065 704 beschrieben. Die Bestandteile des GC-Moduls und deren Zusammenwirken sind darin beschrieben. Entsprechend gilt der Inhalt dieser Schrift als in der vorliegenden Anmeldung aufgenommen. Wie in EP 2 065 704 aufgeführt, wird ein kommerziell erhältliches GC-Modul im Gaschromatograph verwendet. Wie aus der weiteren Darstellung der vorliegenden Erfindung hervorgeht, kann ein solches Chromatographen-Modul nach Bedarf und Anwendungsgebiet angepasst und abgeändert werden.

Für die Gasextraktion wird ein Gasextraktor mit gaspermeabler Membran verwendet, bei dem die Membran mindestens eine Längswand eines Kanals im Gasextraktor bildet. In solchen Gasextraktoren wird Gas und eine Flüssigkeit, aus der Gas extrahiert werden soll, entlang des Kanals und entlang der Membran durch den Extraktor geführt.

Die gaspermeable Membran ist für das oder für die gewünschten Gase, beispielsweise Transformatorengase, durchlässig, vorzugsweise hochdurchlässig. Für diejenige Flüssigkeit, aus der die Gase extrahiert werden sollen, beispielsweise Transformatorenöl, ist sie jedoch gering durchlässig, vorzugsweise undurchlässig.

Solche Extraktoren eignen sich sehr gut für eine Miniaturisierung. Im folgenden sind drei Beispiele für miniaturisierte Gasextraktoren beschrieben, wie sie vorzugsweise in der Vorrichtung gemäss Erfindung verwendet werden.

Mikroreaktoren, in welchen eine Membran jeweils eine Wand eines Kanals im Reaktor bildet, sind sogenannte ,channel by channel microreaktors' oder im Fall von zwei Kanälen, Dual-Kanal-Mikroreatoren. Ein Beispiel eines Dual-Kanal Mikroreaktors ist in der Publikation von Maurya et al. "Continous in situ generation, separation, and reaction of Diazomethane in a Dual-Channel Microreaktor", Angew. Chem. Int. Ed. 2011, 50, 5952ff beschrieben und gezeigt. In einer Ausführungsform der Vorrichtung beinhaltet das Extraktionsmodul mindestens einen Dual-Kanal Mikroreaktor.

Gemäss einer weiteren Ausführungsform der erfindungsgemässen Vorrichtung, weist der mindestens eine Gasextraktor mindestens eine röhrenförmig angeordnete Membran auf. Dabei wird ein Kanal im Extraktor durch die Membran gebildet. Eine Variante eines Gasextraktors mit einer einzelnen röhrenförmigen Membran ist ein sogenannter Tube-in-Tube Extraktor oder Doppelrohr-Extraktor. Eine Gasextraktion geschieht darin über eine rohrförmige, gasdurchlässige, aber nicht flüssigkeitsdurchlässige Membran, die in einem Aussenrohr, beispielsweise einem Metallrohr oder Kunststoffrohr, eingebracht ist. Typischerweise wird eine Flüssigkeit zwischen Membran und Aussenrohr geführt, während ein Extraktionsgas und das extrahierte Gas aus der Flüssigkeit im Inneren der Membran geführt werden. Ein prinzipieller Aufbau eines Doppelrohr-Gasextraktors ist beispielsweise in Koos et al. 'Teflon AF-2400 mediated gas-liquid contact in continuous flow methoxycarbonylations and in-line FTIR measurement of CO concentration', Org. Biomol. Chem., 2011, 9, 6903-6908, anhand eines Doppelrohr-Gasextraktors für einen CO-Austausch über eine spezielle Teflon Membran gezeigt.

Ein weiteres Beispiel eines für die erfindungsgemässe Vorrichtung geeigneten miniaturisierten Gasextraktors, welcher mehrere röhrenförmige Membrane in einem Aussenrohr aufweist, ist ein sogenannter "multiple tubes in a single tube"-Extraktor. Ein Beispiel für einen solchen Extraktor ist das Extraktionsmodul PDMSXA-2500 der Firma MedArray, USA, mit PDMS Membranen, welche diverse solcher Mehrfachrohr-Extraktoren vertreibt.

Für die Extraktion von diversen Gasen haben sich speziell Membranen aus Polydimethylsiloxan (PDMS) und Polytetrafluorethylene (PTFE), letztere allgemein als Teflon bekannt, als geeignet erwiesen. Während Membrane aus PDMS vorwiegend in wässrigen Systemen Anwendung finden, haben sich gewisse Vertreter der amorphen, fluorhaltigen Polymermaterialien aufgrund ihrer Gasdurchlässigkeit und Langlebigkeit als speziell geeignet in der Verwendung zur Extraktion von Transformatorengasen erwiesen. Typische Analyte in der Analyse von Transformatorengase sind H2, N2, 02, CO, CO2, CH4, C2H2, C2H4, C2H6, C3H6, C3H8. Eine im Gasextraktor verwendete Membran ist vorzugsweise für mehrere oder alle dieser Stoffe durchlässig. Werden mehrere Gasextraktoren im Gasextraktionsmodul verwendet, können diese die gleichen Membrane aufweisen oder Membrane, die für unterschiedliche Gase unterschiedlich permeabel sind.

In der Extraktion und Analyse von Transformatorengasen werden bis anhin keine miniaturisierten Gaschromatographen verwenden. Auch sind keine miniaturisierten Gasextraktionsmodule bekannt, welche zudem in Kombination mit miniaturisierten Gaschromatographen verwendet werden. Die Anforderung an die Geräte in solchen Anwendungen sind hoch. Insbesondere ist eine gewisse Robustheit erforderlich, da die Geräte in der Regel wechselnden Umgebungsbedingungen ausgesetzt sind. Dies gilt insbesondere für kontinuierliche online Analysen, da ein Gerät dafür vor Ort installiert ist. Auch sollten die Kosten für solche Geräte nicht zu hoch sein, da ansonsten eine möglichst flächendeckende Installation der Vorrichtungen kaum in Betracht gezogen wird.

Mit der erfindungsgemässen Vorrichtung können Anschaffungs- und Unterhaltskosten gegenüber bekannten Trafogasanalysevorrichtungen welche auf gaschromatographischen Analysemethoden beruhen um vorzugsweise bis zu 50 Prozent gesenkt werden. Ferner ist es mit der erfindungsgemässen Vorrichtung möglich Messresultate zu erhalten, welche mit den internationalen Normen vergleichbar sind, wie sie für die Transformationsgasanalyse beschrieben sind (Bspw.: IEC Norm 60567 Messverfahren, Norm 60599 Interpretation der Messresultate).

Gemäss einem weiteren Aspekt der Erfindung weist der Gaschromatograph ein an der Trägerplatte angebrachten Anschlussblock mit Trägergas- und Probengasanschlüssen zum Anschliessen von Trägergas- und Probengaszufuhrleitungen und Trägergas- und Probengasabfuhrleitungen an die Kapillaren auf. Dabei ist das Gasextraktionsmodul dichtend lösbar direkt an den Anschlussblock des Chromatographenmoduls angeschlossen. Dabei können Probengasauslass des Gasextraktionsmoduls und Anschlussblock über vorzugsweise möglichst kurze Schlauch- und Rohrverbindungen und entsprechende Kupplungen, beispielsweise Verschraubungen, miteinander verbunden sein. Ein Probengasauslass kann jedoch auch direkt, über eine entsprechende Kupplung, beispielsweise einen Adaptor, mit dem Anschlussblock verbunden sein. Zwischen Probengasauslass des Gasextraktionsmoduls und Anschlussblock sind dann keine zusätzlichen Schlauch- oder Rohrverbindungen eingebracht. Dies ermöglicht eine weitere Reduzierung von Totvolumen und die Verbesserung der Präzision einer Messung. Zudem kann ein Übergang und damit eine mögliche Fehlerquelle eliminiert werden. Eine Undichtheit in einem Verbindungsstück, aber auch in einem Schlauch selber, kann damit umgangen werden.

Gemäss einem weiteren Aspekt der erfindungsgemässen Vorrichtung, ist der Probengasauslass des Gasextraktors integral mit einem Verbindungsabschitt der Probengasleitung und diese wiederum integral mit einer daran anschliessenden Kapillare zum Injektor verbunden. Damit ist ein Extraktionsvolumen quasi integral mit dem Injektor verbunden. Eine solch integrale Variante kann beispielsweise derart realisiert werden, dass der mindestens eine Gasextraktor und der Verbindungsabschnitt der Probengasleitung auf einer gemeinsamen Trägerplatte wie das GC-Modul angeordnet sind. Damit bildet das Gaschromatographen-Modul und der mindestens eine Gasextraktor ein integrales Bauteil der Vorrichtung.

Eine solch integrale Lösung erlaubt eine weitere Minimierung des Totvolumens der Vorrichtung. Auch kann ein solch integrales Bauteil einfach ersetzt werden, ohne dass neue und bestehende Mess- und Analyse-Komponenten aneinander angepasst werden müssen. Auch kann eine Kalibration des integralen Bauteils bereits vor Einbau vorgenommen werden.

In der erfindungsgemässen Vorrichtung beträgt ein Totvolumen zwischen Gasextraktionsmodul und Gaschromatograph typischerweise weniger als 500 Mikroliter, vorzugsweise weniger als 200 Mikroliter und beispielsweise weniger als 100 Mikroliter. Beispielsweise liegt ein Totvolumen zwischen 10 Mikroliter und 50 Mikroliter. Wird ein Gasextraktionsmodul dichtend lösbar direkt an einen Anschlussblock des Chromatographen-Moduls angeschlossen oder ein Extraktor integral mit einem GC-Modul ausgebildet, liegt ein Totvolumen im unteren der angegebenen Bereiche von wenigen Mikrolitern oder wenigen zehn Mikrolitern. Generell beschreibt ein Totvolumen das Volumen in einer Probengasleitung ohne Membrankontakt, aber inklusive einem Injektionsvolumen.

Geringe Totvolumen erlauben eine sehr geringe Ansprechgeschwindigkeit der Vorrichtung. Diese kann für die erfindungsgemässe Vorrichtung weniger als 15 min, vorzugsweise weniger als 10 Minuten, beispielsweise weniger als 5 min betragen. Kleine Totvolumen kombiniert mit kleinen Extraktionsvolumen ergeben weniger Fehler aufgrund einer Verdünnung und erlauben eine schnellere Messung bzw. eine schnellere Antwortzeit bis ein Messresultat erhältlich ist. Eine Ansprechzeit kann in Vorrichtungen mit miniaturisieren Komponenten, wie in der erfindungsgemässen Vorrichtung entsprechend kurz gehalten werden.

Gemäss einem weiteren Aspekt der erfindungsgemässen Vorrichtung, weist diese zwei Präzisionsventile auf. Dabei ist mindestens eines der beiden Präzisionsventile dem mindestens einen Gasextraktor vorgeschaltet und vorzugsweise als Mehrwegventil ausgestaltet. In vorteilhaften Ausführungsformen weist die erfindungsgemässe Vorrichtung genau zwei Präzisionsventile auf. In einer einfachen Version können dies einfache Auf-Zu Ventile sein oder beispielsweise zwei Mehrwegventile. Das dem mindestens einen Gasextraktor vorgeschaltete Präzisionsventil dient zur Steuerung der Zuführung von Extraktionsgas in und durch den Gasextraktor. Die Vorrichtung ist mit einer entsprechenden Steuerung, welche unter anderem die Betätigung der Ventile steuert, versehen. Der Begriff 'vorgeschaltet' ist bezüglich der Richtung des Prozessablaufs von Extraktion und Analyse zu verstehen (m.a.W. ein Ventil ist einem Extraktionsgaseinlass in den Extraktor vorgeschaltet).

Im folgenden werden zwei Aufbauversionen und damit verbundenen Funktionsprinzipien der Vorrichtung beschrieben. Die Vorrichtung weist dazu eine Steuerung auf, welche eine entsprechende Steuerung der Ventile erlaubt.

Einseitiger Aufbau: Eine Probengaszuführung in den Injektor ist mit einer ersten Probengaszufuhrkapillare, beispielsweise am Anschlussblock des Chromatographen-Moduls verbunden. Eine Probengaszufuhr in den Injektor wird dabei durch die Steuerung der Präzisionsventile gesteuert, derart, dass Probengas vom Extraktionsmodul durch die erste Probengaszufuhrkapillare zum Injektor führbar ist, derart, dass der Injektor einseitig mit Probengas beaufschlagbar ist.

Zur Injektion des Probengases in den Injektor wird das sich im Injektionsloop befindliche Probengas mit durch das geöffnete Ventil hindurchströmendem Extraktionsgas mit Druck beaufschlagt. Das Extraktionsgas steht dazu unter erhöhtem Druck von vorzugsweise wenigen bar. Das sich in der Probengasleitung befindliche Probengas wird Richtung Injektor geschoben und in den Injektor initiiert. In einer einseitigen Druckbeaufschlagung des Injektors wird ein Probengas im wesentlichen durch die Probengasleitung gespült.

Bei einseitig mit Druck beaufschlagten Injektoren wird ein Gas, beispielsweise Probengas oder Reinigungsgas, generell nur in einer Richtung in den Injektor eingeführt und (nicht initiiertes Gas) in derselben Richtung aus dem Injektor wieder herausgeführt. Eine einseitige Injektion erlaubt eine Injektion mit nur einem Gasextraktor. Es werden weniger Elemente benötigt und ein Probengaseinlass und ein Probengasauslass sind klar zugeordnet. Zudem ist eine einseitige Injektion gut kontrollierbar.

Zweiseitiger Aufbau: Eine Probengaszuführung in den Injektor ist mit einer ersten Probengaszufuhrkapillare und mit einer zweiten Probengaszufuhrkapillare verbunden. Eine Probengaszufuhr in den Injektor wird dabei durch die Steuerung der Präzisionsventile gesteuert, derart, dass Probengas vom Extraktionsmodul durch die erste und durch die zweite Probengaszufuhrkapillare zum Injektor führbar ist, derart, dass der Injektor beidseitig mit Probengas beaufschlagbar ist. Eine Probengasabfuhrkapillare übernimmt dabei die Funktion der zweiten Probengaszufuhrkapillare für den Vorgang der Injektion. Der Injektor wird in dieser Ausführungsform für den Vorgang der Injektion über seinen Einlass und über seinen Auslass und somit beidseitig mit Druck beaufschlagt.

Für eine beidseitige Druckbeaufschlagung ist vorzugsweise ein weiterer Gasextraktor mit gaspermeabler Membran vorgesehen. Dabei ist jedem der beiden Gasextraktoren ein Präzisionsventil, vorzugsweise ein Mehrwegventil, vorgeschaltet. Vorzugsweise ist dann eine Probengasabfuhrleitung des einen Gasextraktors mit der ersten Probengaszufuhrkapillare verbunden und eine Probengasabfuhrleitung des weiteren Gasextraktors ist mit der zweiten Probengaszufuhrkapillare verbunden. Für den Vorgang der Injektion werden beide Ventile geöffnet und Extraktionsgas wird mit Überdruck durch die beiden Extraktoren in Richtung Chromatographen-Modul geleitet. Das sich im Injektionsloop befindliche Probengas wird von zwei Seiten komprimiert und in den Injektor initiiert. Dieser schnelle Vorgang geschieht im wesentlichen durch eine Pfropfenströmung. Bei einer möglichen nachfolgende 'Spülung' wird Extraktionsgas mit Überdruck in den Gaskreislauf geleitet. Das Gas entweicht durch die Membran in die Flüssigkeit. Dieser Vorgang kann mehrmals wiederholt werden.

In beiden Funktionsvarianten kann eine Injektion mit Vakuum, beispielsweise einer Pumpe, unterstützt und verbessert werden, vorzugsweise ohne dass zusätzliche Ventile benötigt werden. Dabei kann an ein Ventil, beispielsweise ein Mehrwegventil, ein Unterdruck angeschlossen sein, durch welchen das Probengas durch die Probengasleitung in Richtung Unterdruck gesaugt wird.

Gemäss einem Aspekt der erfindungsgemässen Vorrichtung beinhaltet die Membran ein amorphes fluorhaltiges Polymer, beispielsweise Polytetrafluorethylen, vorzugsweise Teflon AF-2400 oder Polydimethylsiloxan (PDMS). Membrane aus diesen Materialien zeichnen sich durch eine sehr gute Gaspermeabilität, erstere insbesondere für Transformatorengase, aus, bei entsprechender Beständigkeit und Langlebigkeit. Vorzugsweise werden für den oder die mehreren Gasextraktoren gemäss Erfindung Membrane aus Teflon AF-2400 verwendet.

Um eine Gasdiffusion zu erhöhen und eine Gaslöslichkeit zu verkleinern, kann in der erfindungsgemässen Vorrichtung eine Heizung zum Heizen einer Flüssigkeit vorgesehen sein. Die Flüssigkeit wird vorzugsweise vor Eintritt ins Extraktionsmodul geheizt. Die Heizung wird bevorzugt in einer Flüssigkeitszuführleitung stromaufwärts zum Extraktionsmodul ein- oder angebracht. Damit wird eine Flüssigkeit bereits mit erhöhter Temperatur in und durch den oder die Gasextraktoren geführt.

Gemäss einem weiteren Aspekt der erfindungsgemässen Vorrichtung, weist das Chromatographen-Modul mehr als eine Trennsäule und/oder mehrere Injektoren auf. Bei mehreren Trennsäulen weist vorzugsweise mindestens eine der Trennsäulen eine Belegung der Trennsäule auf, welche zu der oder den Belegungen der weiteren Trennsäulen unterschiedlich ist. Es können auch alle Trennsäulen zueinander unterschiedliche Belegungen aufweisen.

Als Trennsäulen für Gaschromatographen finden typischerweise Dünnschicht oder gepackte Trennsäulen Anwendung. Unterschiedliche Materialen für ein Dünnschicht- oder Packungsmaterial, - für die vorliegenden Zwecke der Einfachheit halber Belegung genannt -, weisen unter anderem unterschiedliche Adsorptionseigenschaften aus. Entsprechend unterschiedliche Trennsäulen weisen unterschiedliche Empfindlichkeiten für unterschiedliche zu analysierende Gase, sowie andere Betriebsverläufe auf. Trennsäulen mit unterschiedlichen Belegungen können somit auf die Analyse gewisser Gase optimiert werden. Auch ist es möglich jeweils eine Trennsäule einem bestimmten Gasextraktor zuzuordnen. Dieser kann dann mit einer entsprechenden Membran versehen sein, welche beispielsweise ebenfalls auf die Extraktion von bestimmten Gasen optimiert ist. Die erfindungsgemässe Vorrichtung kann somit auf erweiterte GC Module (mehrere Trennsäulen, mehrere Injektoren) und erweiterte Extraktionsmodule (mehrere Gasextraktoren) ausgebaut werden. Dabei können die einzelnen Trennsäulen, Injektoren und Gasextraktoren je nach Bedarf seriell oder parallel verschaltet sein. Ein Beispiel für Trennsäulen und ein Packungsmaterial für eine Trennsäule, die für die Verwendung in der Vorrichtung gut geeignet sind, sind flexible, mikrogepackte Trennsäulen, beispielsweise sogenannte HayeSep-Trennsäulen, wie sie von der Firma Hayes Separation Inc., Australien, erhältlich sind.

Gemäss einem weiteren Aspekt der erfindungsgemässen Vorrichtung, weist die Vorrichtung Anschlüsse für die Zu- und Abfuhr eines Kalibrierfluids zum Kalibrieren der Vorrichtung auf. Ein Kalibrierfluid ist beispielsweise eine Kalibrierflüssigkeit oder ein Kalibriergas.

Eine Kalibrierung der Vorrichtung kann prinzipiell mit einer Kalibrierflüssigkeit mit definiertem Gasgehalt vorgenommen werden. Dies hat den Vorteil, dass sämtliche Komponenten, inklusive Extraktor und Trennsäulen auf der Basis der definierten Kalibrierflüssigkeit kalibriert werden können. Diese wird anstelle der ansonsten in der Vorrichtung zu analysierenden Flüssigkeit durch die Vorrichtung geleitet. Dies ergibt direkt (ohne Korrekturfaktoren) einen Kalibrationswert für die Vorrichtung. Wird für die Kalibrierung ein Kalibrationsgas verwendet, so kann dieses auf dem gleichen Weg wie Extraktionsgas durch die Vorrichtung geführt werden. Da jedoch die Permeabilitätseigenschaften einer Membran in der Regel unterschiedlich sind für unterschiedliche Gase, muss bei der Kalibration mit Kalibrationsgas ein Korrekturfaktor entsprechend den unterschiedlichen Gasen mitberücksichtigt werden.

Die erfindungsgemässe Vorrichtung ist speziell geeignet für die Verwendung zur online Extraktion und Analyse von Transformatorengasen aus Transformatorenöl. Sie wird vorzugsweise für die kontinuierliche Analyse des Gasgehalts in Ölen von Leistungstransformatoren verwendet. Die Vorrichtung kann dabei vor Ort installiert und permanent mit einem Transformator verbunden sein. Die erfindungsgemässe Vorrichtung kann jedoch auch in anderen Gebieten Verwendung finden, in denen Gasextraktion aus einer Flüssigkeit und eine möglichst kostengünstige, kontinuierliche Gasüberwachung gewünscht ist. Beispielsweise auf dem Gebiet der Biotechnologie, bei Fermentationsprozessen oder bei der Herstellung von Biogasen. Die erfindungsgemässe Vorrichtung findet beispielsweise auch Verwendung bei der Messung von gasförmigen und tiefsiedenden, flüssigen Analyten, die nicht mit Sensoren gemessen werden können.

Weitere Details und vorteilhafte Ausgestaltungen der Erfindung ergeben sich aus der nachstehenden Beschreibung von Ausführungsbeispielen anhand der Zeichnungen. Es zeigen:
- Fig. 1: eine blockschematische Darstellung der erfindungsgemässen Kombination von Gasextraktionseinheit und Gaschromatograph;
- Fig. 2a,2b: eine schematische Darstellung einer ersten, 'zweiseitigen' Betriebsart der erfindungsgemässen Vorrichtung; im Zustand, in dem Probengas gebildet wird (Fig. 2a) und im Zustand, in dem Injektion erfolgt (Fig. 2b);
- Fig. 3a-3c: eine schematische Darstellung einer zweiten, 'einseitigen' Betriebsart der erfindungsgemässen Vorrichtung; in den Zuständen in dem Probengas gebildet wird (Fig. 3a), in dem Injektion erfolgt (Fig. 3b), und in dem der Injektionsloop gespült wird (Fig. 3c)
- Fig. 4: eine vereinfachte technische Zeichnung einer Aufsicht in ein Gehäuse in dem die erfindungsgemässe Vorrichtung eingebaut ist;
- Fig. 5: einen Schnitt entlang der Linie A-A in Fig. 4;
- Fig. 6: eine schematische Skizze einer Schnittansicht durch einen Doppelrohr-Gasextraktor mit gaspermeabler Membran;
- Fig. 7: ein Blockschaltbild für eine Vorrichtung beispielsweise gemäss Fig. 1 mit zusätzlichen Kalibriergasanschlüssen.

Ausser es ist in der Anmeldung anderweitig beschrieben oder es sind für eine Fachperson abweichende Angaben offensichtlich, gelten die folgenden Definitionen:
Probengasleitung: Gasvolumen zwischen den zwei Ventilen;
Totvolumen: Volumen der Probengasleitung ohne Membrankanalvolumen im Gasextraktor;
Injektionsloop: Totvolumen der Probengasleitung auf dem GC-Modul;
Injektionsvolumen: Probengasleitung im Injektor vor Injektion.

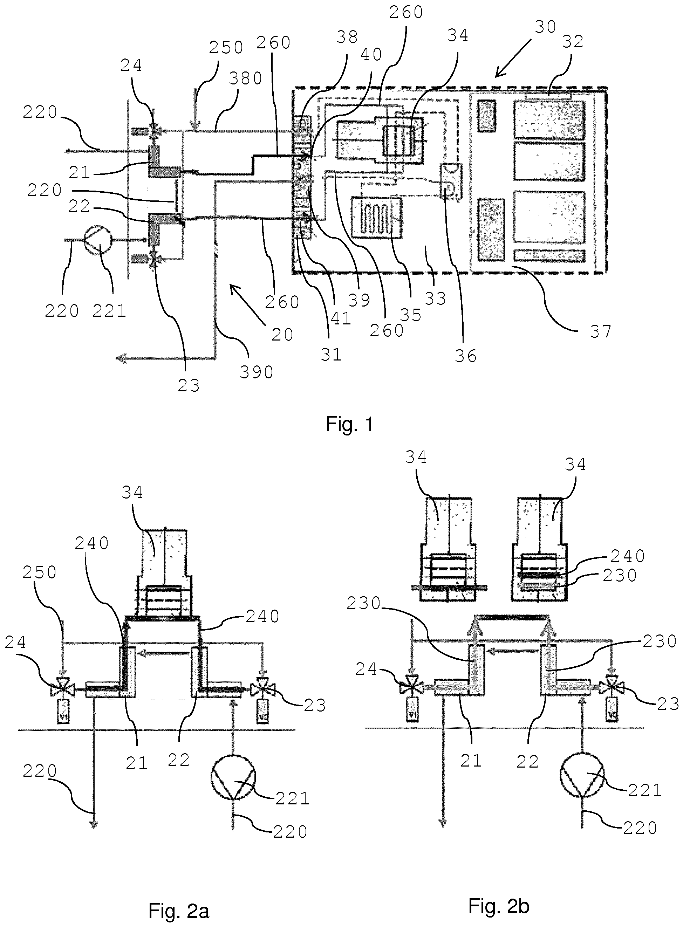

Fig. 1 zeigt schematisch ein kombiniertes Gasextraktionsmodul 20 und Chromatographen-Modul 30 mit einem Gas-Anschlussblock 31. Das zentrale Bauelement des Gaschromatographen ist das miniaturisierte Chromatographen-Modul 30. Dieses ist auf einer Trägerplatte 33 aufgebaut, auf der alle Kernkomponenten eines Gaschromatographen enthalten sind. Es sind dies ein elektrisch gesteuerter Injektor 34, eine mit einer Heizung versehene Dünnschicht- oder gepackte Trennsäule 35 und ein ggf. auch beheizbarer Wärmeleitfähigkeitsdetektor 36 sowie eine Mikroprozessor-Elektronik 37 zur Steuerung des Chromatographen-Moduls 30 sowie zur Auswertung, Speicherung und Übermittlung der Messdaten über die Kommunikationsschnittstelle 32. Ferner sind auf der Trägerplatte 33 diverse Kapillaren vorgesehen, welche einerseits den Injektor, die Trennsäule und den Detektor verbinden und anderseits zum Anschlussblock 31 führen. Die in den Anschlussblock 31 eingesetzten vier Endabschnitte der Kapillaren sind Trägergaszufuhrkapillare 38, Trägergasabfuhrkapillare 39, erste Probengaszufuhrkapillare 40 und zweite Probengaszufuhrkapillare 41, wobei die zweite Probengaszufuhrkapillare 41 gleichzeitig auch als Probengasabfuhrkapillare dient. Dieselben Referenzeichen werden entsprechend auch für die Anschlüsse im Anschlussblock 31 für eine Trägergaszufuhr 38, für eine Trägergasabfuhr 39, eine Probengaszufuhr 40 und eine Probengasabfuhr 41 verwendet.

Alle zentralen Betriebsfunktionen des Chromatographen-Moduls 30 selbst können von der Mikroprozessor-Elektronik 37 autonom gesteuert werden, wobei übergeordnete Steuerbefehle von einem Rechner (nicht gezeigt) übernommen und abgearbeitet werden können. Die Mikroprozessor-Elektronik 37 kann auch mit Schalt-Ausgängen zur Ansteuerung von modul-externen Komponenten wie z.B. Ventilen und Pumpen ausgestattet sein.

Das Chromatographen-Modul 30 ist im Dokument EP 1 588 156 in allen Details beschrieben, so dass der Fachmann diesbezüglich keiner näheren Erläuterung bedarf. Die folgenden Ausführungen beschränken sich daher auf die an die Kopplung mit dem Extraktionsmodul 20 angepassten Komponenten der erfindungsgemässen Vorrichtung.

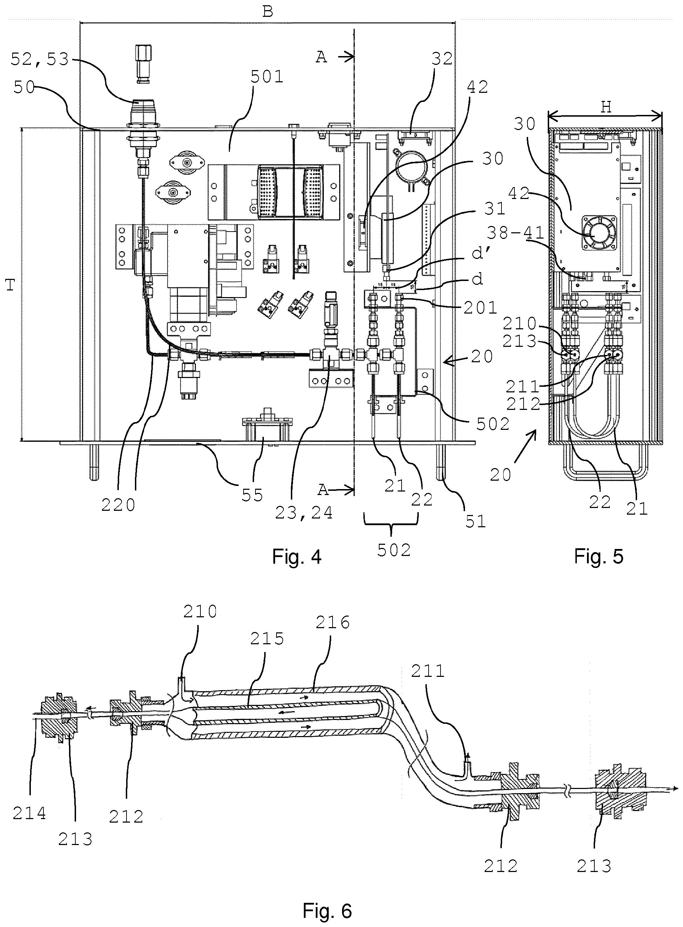

Die zentralen Bauteile des Extraktionsmoduls 20 sind in dieser Ausführungsform zwei Doppelrohr-Membranextraktoren 21, 22, wobei die Membran vorzugsweise ein aus Teflon AF 2400 hergestellte Membran ist, wie sie von der Firma Du Pont (Wilmington, Delaware, USA) erhältlich ist. Der schematische Aufbau eines solchen Doppelrohr-Membranextraktors ist anhand Fig. 6 gezeigt und wird weiter unten beschrieben. Eine für den Öltransport vorgesehene Transformatorenölleitung 220 (Fliessrichtung mit Pfeilen eigezeichnet), zweigt zu analysierendes Öl von einem Transformator (nicht gezeigt) ab. Die Leitung 220 führt das Öl über einen Öleinlass in und durch den einen Extraktor 22, dann in und durch den zweiten Extraktor 21, durch einen Ölauslass aus dem zweiten Extraktor und aus dem Extraktionsmodul hinaus und wieder zurück in den Transformator. In der Transformatorenleitung 220 ist eine Pumpe 221 vorgesehen zum kontinuierlichen, vorzugsweise gleichmässigen Transport von Transformatorenöl durchs Extraktionsmodul 20. Typische Öldurchflussmengen sind im Bereich von einigen Millilitern pro Minute (ml/min), beispielsweise im Bereich von 1 ml/min bis 30 ml/min, vorzugsweise im Bereich von 2 ml/min bis 20 ml/min.

Beiden Extraktoren 21,22 ist je ein Zweiweg-Ventil 23,24 vorgeschaltet. Eine Gasquelle (nicht gezeigt), beispielsweise eine Edelgas-Druckflasche, ist über ein Druckreduzierventil mit den Zweiweg-Ventilen verbunden. Die Gasquelle beinhaltet das Trägergas, beispielsweise Argon, welches vorzugsweise gleichzeitig zur Extraktion verwendet wird. Das Trägergas wird direkt in den Trägergaseinlass 38 und Trägergasauslass durch das GC-Modul 30 geleitet (Trägergasfluss in 380, out 390). Zur Extraktion wird das Gas 250 über die Ventile 23,24 in die Extraktoren 21,22 geleitet. Ein Gaseinlass geschieht vorzugsweise unter einem bestimmten Druck, beispielsweise zwischen 1.5 bar bis 6 bar, vorzugsweise 2 bar bis 4 bar.

Wie schon weiter vorne erläutert, ist es von besonderer Wichtigkeit, dass wenigstens die Probengas führenden Teile der Anordnung möglichst totvolumenfrei konzipiert sind. Dies wird durch die spezielle Anordnung von Extraktoren 21,22 und Anschlussblock 31 erreicht, an welchen alle gasführenden Leitungen direkt oder mittels kurzen Schlauchverbindungen, ans Chromatographen-Modul 30 angeschlossen sind. Dies ermöglicht eine allgemeine Minimierung des Verbindungsabschnitts 260 der Probengasleitung, welcher sich vom Ausgang Gasextraktor 21 über den Injektor 34 und zum Ausgang des zweiten Gasextraktors 22 erstreckt. Die gesamte Probengasleitung erstreckt sich im wesentlichen über die Strecke zwischen den zwei Ventilen 23,24.

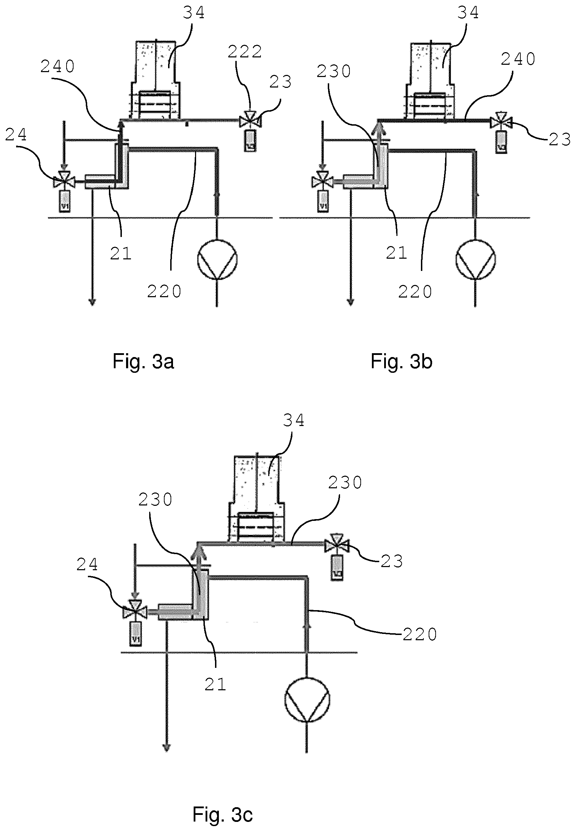

Die nahe Kopplung ermöglicht eine spezielle Wechselwirkung der Module, wie sie anhand der folgenden zwei Funktionsprinzipien gemäss den Figuren 2a,2b und 3a,3b und 3c gezeigt ist.

In den **Fig. 2a und 2b** ist ein Funktionsprinzip der erfindungsgemässen Vorrichtung, beispielsweise gemäss Fig. 1 gezeigt. Darin wird der Injektor 34 zur Injektion von Probengas zweiseitig mit Druck beaufschlagt. Gleiche oder ähnliche Elemente der Vorrichtung sind entsprechend mit den gleichen Referenzeichen versehen. Der Übersichtlichkeit halber ist nur der Injektor des Chromatographen-Moduls 30 gezeigt. In Fig. 2a ist die Probengasbildung im Extraktionsvolumen der beiden Extraktoren 21,22 dargestellt. Durch Diffusion verteilt sich das aus der Flüssigkeit gelöste und durch die Membran transferierte Probengas 240 aufgrund der kurzen Wege bis in den und im Injektionsloop. Bei diesem Prozess sind die beiden Ventile 23,24 für die Extraktionsgaszuführung geschlossen. Der Gaskreislauf wurde zuvor mit Gas 250, beispielsweise Argon, unter Druck geflutet. Das Gas wird dabei mit leicht erhöhtem Druck, beispielsweise 2bar, in den Kreislauf eingeführt. Nach Schliessen der Ventile 23,24 baut sich Argon durch die Membran in den Extraktoren 21,22 bis auf Umgebungsdruck ab. Gemäss den Partialdrücken wird entsprechend Transformatorengas in der Gegenrichtung durch die Membran transportiert. Ein solcher Gastransport dauert in der Regel wenige Minuten und beträgt je nach gewünschtem Messzyklus ca. 2 bis 15 Minuten, beispielsweise 3 bis 10 Minuten. Nach einer gewissen Zeit, in der sich Probengas angesammelt hat werden die Ventile 23,24 geöffnet. Wie in Fig. 2b gezeigt, komprimiert das von zwei Seiten einschiessende, mit Druck beaufschlagte Argon das Probengas zweiseitig (Pfeile 230). Das Extraktionsgas drängt das Probengas weiter in den Injektionsloop und Injektion in den Injektor 34 erfolgt. Oben links in Fig. 2b ist der Injektor nach der Druckbeaufschlagung, aber gerade noch vor der Injektion gezeigt. Die Fig. 2b, oben rechts zeigt den Injektor 34 nach der Injektion. Im 'unteren' Injektionsloop fliesst Extraktionsgas, welches nach Schliessen der Ventile 23, 24 zum Spülen des Gaskreislauf genutzt werden kann.

Der Injektionsloop erstreckt sich in dieser Ausführungsform vom Anschlussblock 31 des Chromatographen-Moduls 30, über entsprechende Kapillaren zum Injektor, durch den Injektor hindurch und über entsprechende Kapillaren wieder zurück zum Anschlussblock. Beispielhafte Volumen für den Injektionsloop sind 15 bis 40 Mikroliter, beispielsweise zwischen 20 und 30 Mikroliter, z.B. 25 Mikroliter. Ein Injektionsvolumen selber beträgt in miniaturisierten Chromatographen-Modulen in der Regel wenige Mikroliter, beispielsweise etwa 0.5 bis 10 Mikroliter. Im Beispiel von Fig. 2b wird der gesamte Gaskreislauf, der beispielsweise in etwa 200-250 Mikroliter beträgt, unter dem Druck des einströmenden Gases bei 2 bar auf die Hälfte komprimiert, bis auf etwa 100 bis 125 Mikroliter.

In den **Fig. 3a, 3b und 3c** ist ein weiteres Funktionsprinzip der erfindungsgemässen Vorrichtung gezeigt, in dem der Injektor 34 zur Injektion von Probengas 240 jedoch nur einseitig mit Druck beaufschlagt wird. Für gleiche oder ähnliche Komponenten werden wiederum dieselben Referenzzeichen verwendet. Das Extraktionsmodul 20 weist nur einen Extraktor 21 auf. Das zweite Ventil 23 ist direkt oder über eine entsprechende Schlauchverbindung mit dem Probengasauslass 40 des Chromatographen-Moduls 30 verbunden. Die Probengasbildung in Fig. 3a verläuft im wesentlichen gleich wie in Fig. 2a beschrieben, mit dem Unterschied, dass Gasdiffusion in den Injektionsloop nur einseitig vom einzigen Extraktor 21 her erfolgt. Gemäss Fig. 3b wird das dem Extraktor 21 vorgeschaltete Ventil 24 geöffnet. Ventil 23, dem Injektor in Probengasfliessrichtung nachgeschaltet, bleibt für den Injektionsvorgang geschlossen. Dies ist in Fig. 3b gezeigt. Nach Öffnen des Ventils 24 strömt Extraktionsgas 230 mit Überdruck durch den Extraktor 21. Probengas 240 wird komprimiert und in Richtung Injektor 34 und Ventil 23 geschoben, wobei Injektion erfolgt. In Fig. 3c ist der nachfolgende Spülvorgang des Gaskreislaufs gezeigt. Dazu sind beide Ventile 23,24 geöffnet und der gesamte Gaskreislauf durch den Extraktor 21 und den Injektor 34 werden von Extraktionsgas 230 durchflossen. Anschliessend an den Spülvorgang wird Ventil 23 wieder geschlossen und der Gaskreislauf hat Umgebungsdruck.

Zur Unterstützung eines Gastransports durch die Probengasleitung kann die Leitung über das Ventil 23 an einen Unterdruck angeschlossen werden. Dies kann durch ein mit dem Ventil 23 verbundene Pumpe 222 geschehen. Eine Unterstützung des Probengastransports ist anhand des einseitigen Funktionsprinzips der Fig. 3a gezeigt, ist jedoch auch für das zweiseitige Funktionsprinzip der Vorrichtung entsprechend anwendbar.

Die technische Zeichnung gemäss **Fig. 4** zeigt eine Aufsicht in ein Gehäuse 50, beispielsweise ein Einschubmodul für ein Rack, in welchem Gehäuse 50 die erfindungsgemässe Vorrichtung einfach und platzsparend eingebaut ist. Der Übersichtlichkeit halber sind vor allem die Hauptkomponenten der Vorrichtung abgebildet. Das Gasextraktions-Modul 20 und das Chromatographen-Modul 30 sind sehr kompakt in einem kleinen Bereich 502 des Gehäuses 50 untergebracht. Zu sehen sind die nahe Anordnung der Gasauslässe 201 des Extraktionsmoduls 20 zu den Gaseinlässen und Gasauslässen 38,39,40,41 im Chromatographen-Modul 30. Die Distanzen d, d' zwischen den Gaseinlässen und Gasauslässen der beiden Module 20,30 in gegenüberliegender und seitlicher Richtung betragen in der Regel lediglich ca. 1-10cm, vorzugsweise 1-5cm, in Fig. 4 beispielsweise 1-2cm. Die kurzen dafür benötigen Schlauchverbindungen sind nicht eingezeichnet.

Die Steuerventile 23,24 für die Extraktoren, sowie die Transformatorenölleitungen 220 sind ausserhalb der eigentlichen Module 20,30 angeordnet. Ausserhalb des Bereichs 502 sind zudem weitere Komponenten wie Heizung, Lüftung, Sensoren zu Überwachung der Vorrichtung oder Bestandteilen davon untergebracht.

Sämtliche Elemente sind vorzugsweise auf einer Gehäuseplatte 501 montiert. Das Gehäuse 50 ist mit Griffen 51 und diversen Anschlüssen für die Zu-und Abführung von zu analysierender Flüssigkeit 52,53, Zu-und Abfuhr von Gasen, Stromversorgung und Datenanschlüssen 32 versehen. Das Gehäuse 50 weist zudem Anzeigen 55 auf, auf welchen aktuelle Angaben oder Einstellungen eines Prozesses oder einzelner Komponenten angezeigt werden können.

In der beispielhaften Ausführungsform gemäss Fig. 4 weist das Gehäuse eine Grösse von ca. 13cm x 50cm x 36cm (HxBxT) auf.

In **Fig. 5** ist die Seitenansicht auf den Bereich 502 des Einschubmoduls gemäss Fig. 4 entlang der Linie A-A gezeigt. Zu sehen sind die bogenförmigen Doppelrohr-Gasextraktoren 21,22 mit Flüssigkeitsein- und -auslässen, sowie Gaszuführungen 210,211,212,213. Das GC-Modul 30 ist mit einem Kühlelement 42, beispielsweise einem Ventilator zur Kühlung des Moduls versehen.

**Fig. 6** zeigt sehr schematisch eine Schnittansicht durch einen bogenförmigen Doppelrohr-Extraktor. Eine Membran 215, beispielsweise aus Teflon, ist röhrenförmig und möglichst zentrisch innerhalb eines Aussenrohrs 216 eingebracht. Das Aussenrohr ist vorzugsweise aus Metall, beispielsweise rostfreiem Stahl, gefertigt. Es kann je nach Anwendung auch aus einem flexibleren Material, wie beispielsweise Kunststoff gefertigt sein. Dieser ist dann vorzugsweise inert gegenüber den im Extraktor verwendeten Flüssigkeiten und Gasen. Im Falle von bogenförmigen Extraktoren, kann eine einzelne zusammenhängende Membran entlang der U-form des Aussenrohrs eingebracht sein. Vorzugsweise sind zwei schlauchförmige Membrane 215 entlang der beiden Beine des Aussenrohrs 216 angeordnet. Der Extraktor weist an seinem einen Ende einen Flüssigkeitseinlass 210 und an seinem anderen Ende einen Flüssigkeitsauslass 211 auf. Der Innenraum der Membran ist über einen Schlauch 214 mit dem Anschlussblock 31 des GC-Moduls verbunden. Der Extraktor weist dazu geeignete Verbindungsstücke 212,213, beispielsweise Verschraubungen auf.

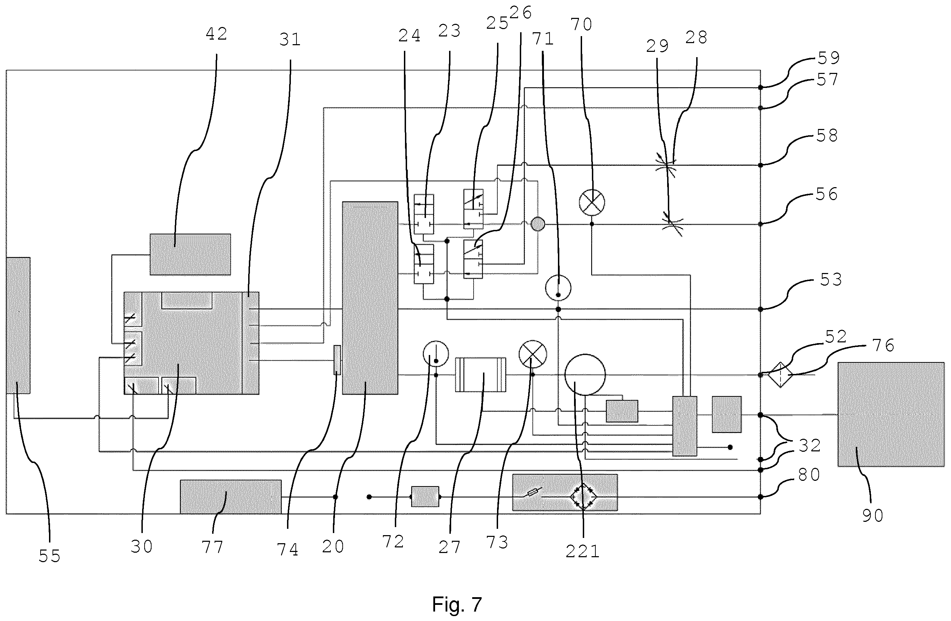

**Fig. 7** zeigt ein Blockschaltbild für eine erfindungsgemässe Vorrichtung beispielsweise gemäss Fig. 1 oder Fig. 4. Gleiche Element sind entsprechend mit gleichen Bezugszeichen versehen. Die Ausführungsform wie in Fig. 7 blockschematisch gezeigt, weist jedoch einen Kalibrierkreislauf und zusätzliche Elemente auf, wie sie in der erfindungsgemässen Vorrichtung eingebracht werden können. Für die integrierte Kalibrierung sind entsprechende Anschlüsse für eine Zuführung 58 und eine Abfuhr 59 von Kalibriergas vorgesehen. Der Kalibriergaskreislauf weist ein Druckreduzierventil 28 im Einlassbereich auf. Vorgeschaltet zum Gasextraktor 20 sind parallel zu den Mehrwegventilen 23, 24 je ein Ventil 25,26 für die Steuerung von Kalibriergas vorgesehen.

Des Weiteren sind im und am Gehäuse diverse Sensoren und Elemente wie folgt vorgesehen:
**52,53** Öleinlass und -auslass; **70** Gasdruckmessung des Trägergases nach Druckreduzierventil **29** für Trägergas; **71** Öltemperaturmessung nach Extraktor; **72** Öltemperaturmessung vor Extraktor; **73** Öldruckmessung vor Extraktor; **27** Heizung für Öl vor Extraktor; **221** Pumpe für Ölkreislauf; **76** Filter in Öleinlass (Schutz vor Verschmutzung des Extraktors); **74** Optischer Sensor für Probengas nach Extraktor (Prüfen auf Ölreste in Probengas zum Schutz des GC Moduls); **32** Interface für Datenanschluss; **90** externes Eingabemodul (z.B. Rechner, Touchscreen); **80** Stromanschluss; **55** Display zur Anzeige aktueller Prozesse und Einstellungen; **42** Lüfter für GC-Modul 30; **77** Gehäuselüftung (z.B. Ventilator); **56,57** Trägergaseinlass und -auslass.

Im folgenden sind Werte und Grössenangaben für beispielhafte Ausführungsformen der erfindungsgemässen Vorrichtung angegeben. Diese dienen lediglich als Beispiel und sind nicht darauf zu beschränken.
- Membran: Teflon AF2400; Durchmesser 0.1016cm; Wandstärke 0.01016cm; Länge der Kontaktfläche Öl-Membran ca. 10cm bis 30 cm pro Einheit; Volumen Extraktionseinheit 0.64ml/100cm, typischerweise 0.2-0.4ml;
- Menge Probengas für Gasanalyse im GC-Modul: 0,5 bis 10µl;
- Totvolumina zwischen Extraktion und Injektor: ca. 0.014ml (pro Extraktor), ca. 30 Mikroliter total; Injektorvolumen 2-8 Mikroliter; Volumen Injektionsloop: 10-100 Mikroliter;
- Extraktionsvolumen: 400 Mikroliter;
- Probegasvolumen: ca. 430 Mikroliter (Extraktionsvolumen (400µl) + Totvolumen (30 µl) + Injektorvolumen (8 µl)).
- Öl: kontinuierlicher Ölfluss; Ölfluss 2.1 bis 15.4 ml/min; Temperatur Raumtemperatur bis 70°C
- Intervall Injektionszyklen (Messzyklen): 1 bis 10 min, typischerweise 1-3 min; Das Heizen und Auskühlen einer Trennsäule dauert je ca. 30 min, während die Messung ca. 2 min dauert; Die Trennsäule wird dabei von ca. 150°C auf 40°C runtergekühlt;
- Zwei Zweiwegventile; ein Druckreduzierer für Trägergas; ev. ein zusätzlicher Druckreduzierer für Kalibriergas, dann ev. ein weiteres Ventil vor Extraktor oder Ausbildung der Zweiwegventile als Dreiwegventile zur Steuerung des Einlass von Kalibriergas.

## Patentansprüche

1. Vorrichtung zum Extrahieren und Analysieren von Gas, aufweisend:
- ein Gaschromatograph mit einem miniaturisierten Chromatographen-Modul (30), welches auf einer Trägerplatte (33) angeordnet einen Injektor (34), eine mit einer Heizung verbundene Trennsäule (35), einen Detektor (36), sowie mehrere Kapillaren zum Verbinden dieser Funktionsteile zur Zu- und Abfuhr von Trägergas und Probengas (240) aufweist;
- ein miniaturisiertes Gasextraktionsmodul (20) mit mindestens einem Gasextraktor (21,22) mit einer gaspermeablen Membran (215) zum Extrahieren von Gas aus einer Flüssigkeit durch die Membran, wobei die Membran mindestens eine Längswand eines Kanals im Gasextraktor bildet, wobei das Gasextraktionsmodul mehrere Anschlussleitungen zum Verbinden des Gasextraktionsmoduls mit einer Flüssigkeitszufuhr und -abfuhr, sowie mit einer Extraktionsgaszufuhr und mit einer Extraktions- und Probengasabfuhr aufweist,
wobei eine Probengasabfuhrleitung des Gasextraktionsmoduls mit einer Probengaszufuhrkapillare (40,41) des Chromatographen-Moduls zur Zufuhr von Probengas verbunden ist und einen Verbindungsabschnitt einer Probengasleitung bildet,
und wobei der Verbindungsabschnitt der Probengasleitung sich vom Gasextraktionsmodul (20) zum Injektor (34) des Chromatographen-Moduls (30) erstreckt und ventilfrei ist.

2. Vorrichtung nach Anspruch 1, wobei das Gaschromatographen-Modul ein an der Trägerplatte (33) angebrachten Anschlussblock (31) mit Trägergas- und Probengasanschlüssen (38,39,40,41) zum Anschliessen von Trägergas- und Probengaszufuhrleitungen und Trägergas- und Probengasabfuhrleitungen an die Kapillaren aufweist, und wobei
das Gasextraktionsmodul (20) dichtend lösbar direkt an den Anschlussblock (31) des Chromatographenmoduls (30) angeschlossen ist.

3. Vorrichtung nach Anspruch 1, wobei der mindestens eine Gasextraktor (21,22) und der Verbindungsabschnitt (260) der Probengasleitung auf der Trägerplatte (33) angeordnet sind, derart, dass Gaschromatographen-Modul (30) und der mindestens eine Gasextraktor (21,22) ein integrales Bauteil der Vorrichtung bilden.

4. Vorrichtung nach einem der vorhergehenden Ansprüche, wobei ein Totvolumen zwischen Gasextraktionsmodul (20) und Gaschromatograph weniger als 500 Mikroliter, vorzugsweise weniger als 200 Mikroliter, beispielsweise weniger als 100 Mikroliter beträgt und beispielsweise zwischen 10 Mikroliter und 50 Mikroliter liegt.

5. Vorrichtung nach einem der vorhergehenden Ansprüche, wobei eine Ansprechzeit kleiner als 15 min, vorzugsweise kleiner als 10 Minuten, beispielsweise kleiner als 5 min beträgt.

6. Vorrichtung nach einem der vorhergehenden Ansprüche, wobei die gaspermeable Membran (215) des mindestens einen Gasextraktors (21,22) mindestens eine röhrenförmig angeordnete Membran ist und der mindestens eine Gasextraktor (21,22) vorzugsweise ein Doppelrohr-Gasextraktor oder ein Mehrfachrohr-Gasextraktor ist.

7. Vorrichtung nach einem der Ansprüche 1-5, wobei der Gasextraktor ein Dual-Kanal Mikroreaktor ist.

8. Vorrichtung nach einem der vorhergehenden Ansprüche, mit zwei Präzisionsventilen (23,24), wobei mindestens eines der beiden Präzisionsventile dem mindestens einen Gasextraktor (21,22) vorgeschaltet ist.

9. Vorrichtung nach Anspruch 8, mit einer Steuerung für die Präzisionsventile (23,24), wobei eine der mehreren Kapillaren eine erste Probegaszufuhrkapillare (40) ist und eine Probengaszuführung in den Injektor (34) mit der ersten Probengaszufuhrkapillare (40) verbunden ist, und wobei eine Probengaszufuhr in den Injektor durch eine Steuerung der Präzisionsventile derart gesteuert ist, dass Probengas (240) vom Extraktionsmodul (20) durch die erste Probengaszufuhrkapillare (40) zum Injektor (34) führbar ist, derart, dass der Injektor einseitig durch die erste Probengaszufuhrkapillare (40) mit Probengas beaufschlagbar ist.

10. Vorrichtung nach Anspruch 8, mit einer Steuerung für die Präzisionsventile (23,24), wobei eine der mehreren Kapillaren eine erste Probengaszufuhrkapillare (40) und eine weitere der mehreren Kapillaren eine zweite Probengaszufuhrkapillare (41) ist und eine Probengaszuführung in den Injektor (34) mit der ersten Probengaszufuhrkapillare (40) und mit der zweiten Probengaszufuhrkapillare (41) verbunden ist, und wobei eine Probengaszufuhr in den Injektor durch eine Steuerung der Präzisionsventile derart gesteuert ist, dass Probengas (240) vom Extraktionsmodul (20) durch die erste und durch die zweite Probengaszufuhrkapillare (40,41) zum Injektor (34) führbar ist, derart, dass der Injektor zweiseitig durch die erste und zweite Probengaszufuhrkapillare (40,41) mit Probengas beaufschlagbar ist.

11. Vorrichtung nach Anspruch 10, mit einem weiteren Gasextraktor (21,22) mit gaspermeabler Membran, wobei beide Präzisionsventile (23,24) je einem der beiden Gasextraktoren vorgeschaltet sind und vorzugsweise Mehrwegventile sind, und wobei eine Probengasabfuhrleitung des einen Gasextraktors (21) mit der ersten Probengaszufuhrkapillare (40) verbunden ist und eine Probengasabfuhrleitung des weiteren Gasextraktors (22) mit der zweiten Probengaszufuhrkapillare (41) verbunden ist.

12. Vorrichtung nach einem der vorhergehenden Ansprüche, wobei die Membran (215) ein amorphes fluorhaltiges Polymer beinhaltet, beispielsweise Polytetrafluorethylen, vorzugsweise Teflon AF 2400, oder Polydimethylsiloxan (PDMS) beinhaltet.

13. Vorrichtung nach einem der vorhergehenden Ansprüche, mit einer Heizung (27) zum Heizen der Flüssigkeit vor Eintritt ins Extraktionsmodul (20).

14. Vorrichtung nach einem der vorhergehenden Ansprüche, wobei das Chromatographen-Modul (30) mehr als eine Trennsäule (35) aufweist, wobei mindestens eine der Trennsäulen eine Belegung der Trennsäule aufweist, welche zur der oder den Belegungen der weiteren Trennsäulen unterschiedlich ist.

15. Vorrichtung nach einem der vorhergehenden Ansprüche, mit Anschlüssen für eine Zu- und Abfuhr eines Kalibrierfluids zum Kalibrieren der Vorrichtung.

16. Verwendung der Vorrichtung nach einem der vorhergehenden Ansprüche zur online Extraktion und Analyse von Transformatorengasen aus Transformatorenöl.

## Claims

1. Apparatus for extracting and analysing gas, comprising:
- a gas chromatograph with a miniaturized chromatograph module (30), which comprises, arranged on a carrier plate (33), an injector (34), a separating column (35) connected to a heater, a detector (36) and a plurality of capillaries for connecting these functional parts for the supply and discharge of carrier gas and sample gas (240);
- a miniaturized gas extraction module (20) comprising at least one gas extractor (21, 22) having a gas-permeable membrane (215) for extracting gas from a liquid through the membrane, the membrane forming at least one longitudinal wall of a channel in the gas extractor, wherein the gas extraction module comprises a plurality of connection lines for connecting the gas extraction module to a liquid supply and discharge, and to an extraction gas supply and to an extraction and sample gas discharge,
wherein a sample gas discharge line of the gas extraction module is connected to a sample gas supply capillary (40,41) of the chromatograph module for supplying sample gas and forms a connecting section of a sample gas line, and wherein the connecting section of the sample gas line extends from the gas extraction module (20) to the injector (34) of the chromatograph module (30) and is valve free.

2. Apparatus according to claim 1, wherein the gas chromatograph module comprises a connection block (31) mounted to the carrier plate (33), the connection block having carrier gas and sample gas connections (38, 39, 40, 41) for connecting carrier gas and sample gas supply lines and carrier gas and sample gas discharge lines to the capillaries, and wherein
the gas extraction module (20) is directly connected to the connection block (31) of the chromatograph module (30) in a sealed detachable manner.

3. Apparatus according to claim 1, wherein the at least one gas extractor (21, 22) and the connecting section (260) of the sample gas line are arranged on the carrier plate (33) such that the gas chromatograph module (30) and the at least one gas extractor (21, 22) form an integral component of the apparatus.

4. Apparatus according to any one of the preceding claims, wherein a dead volume between gas extraction module (20) and gas chromatograph is less than 500 microliters, preferably less than 200 microliters, for example less than 100 microliters, and is for example between 10 microliters and 50 microliters.

5. Apparatus according to any one of the preceding claims, wherein a response time is less than 15 minutes, preferably less than 10 minutes, for example less than 5 minutes.

6. Apparatus according to any one of the preceding claims, wherein the gas-permeable membrane (215) of the at least one gas extractor (21,22) is at least one tubularly arranged membrane and the at least one gas extractor (21,22) is preferably a double-tube gas extractor or a multiple-tube gas extractor.

7. Apparatus according to any one of claims 1-5, wherein the gas extractor is a dual-channel micro reactor.

8. Apparatus according to any one of the preceding claims, comprising two precision valves (23, 24), wherein at least one of the two precision valves is connected upstream of the at least one gas extractor (21, 22).

9. Apparatus according to claim 8 comprising a controller for the precision valves (23,24), wherein one of the plurality of capillaries is a first sample gas supply capillary (40) and a sample gas supply into the injector (34) is connected to the first sample gas supply capillary (40), and wherein a sample gas supply into the injector is controlled by a control of the precision valves such that sample gas (240) is guidable from the extraction module (20) through the first sample gas supply capillary (40) to the injector (34) such that sample gas can be applied through the first sample gas supply capillary to one side of the injector (40).

10. Apparatus according to claim 8, comprising a controller for the precision valves (23, 24), wherein one of the plurality of capillaries is a first sample gas supply capillary (40) and another of the plurality of capillaries is a second sample gas supply capillary (41), and wherein a sample gas supply into the injector (34) is connected to the first sample gas supply capillary (40) and to the second sample gas supply capillary (41), and wherein a sample gas supply into the injector is controlled by a control of the precision valves such that sample gas (240) is guidable from the extraction module (20) through the first and through the second sample gas supply capillary (40, 41) to the injector (34) such that sample gas can be applied through the first and second sample gas supply capillaries (40, 41) to two sides of the injector.

11. Apparatus according to claim 10, comprising a further gas extractor (21, 22) having a gas-permeable membrane, wherein both precision valves (23, 24) are each connected upstream of one of the two gas extractors and preferably are multiway valves, and wherein a sample gas discharge line of one of the gas extractors (21) is connected to the first sample gas supply capillary (40) and a sample gas discharge line of the further gas extractor (22) is connected to the second sample gas supply capillary (41).

12. Apparatus according to any one of the preceding claims, wherein the membrane (215) comprises an amorphous fluorine-containing polymer, for example polytetrafluoroethylene, preferably Teflon AF 2400, or polydimethylsiloxane (PDMS).

13. Apparatus according to any one of the preceding claims, comprising a heater (27) for heating the liquid before entering the extraction module (20).

14. Apparatus according to any one of the preceding claims, wherein the chromatograph module (30) comprises more than one separating column (35), wherein at least one of the separating columns has an occupation of the separating column which is different from the occupation or the occupations of the further separating columns.

15. Apparatus according to any one of the preceding claims, comprising connections for a supply and discharge of a calibration fluid for calibrating the apparatus.

16. Use of the apparatus according to any one of the preceding claims for the on-line extraction and analysis of transformer gases from transformer oil.

## Revendications

1. Dispositif d'extraction et d'analyse de gaz comprenant :
- un dispositif de chromatographie en phase gazeuse ayant un module de chromatographie miniaturisé (30) qui comporte, montés sur une plaque de support (33), un injecteur (34), une colonne de séparation (35) reliée à un chauffage, un détecteur (36) ainsi que plusieurs capillaires permettant la liaison de cet emplacement fonctionnel pour permettre l'entrée et la sortie de gaz porteur et de gaz échantillon (240),
- un module d'extraction de gaz miniaturisé (20) ayant au moins un dispositif d'extraction de gaz (21, 22) muni d'une membrane perméable aux gaz (215) permettant d'extraire des gaz d'un liquide au travers de la membrane, la membrane formant au moins une paroi longitudinale d'un anal situé dans le dispositif d'extraction des gaz, le module d'extraction des gaz comportant plusieurs conduites de raccordement permettant de relier le module d'extraction des gaz à une entrée et à une sortie de liquide ainsi qu'à une entrée de gaz d'extraction et à une sortie de gaz d'extraction et de gaz échantillon,
une conduite de sortie de gaz échantillon du module d'extraction de gaz étant reliée à un capillaire d'entrée de gaz échantillon (40, 41) du module de chromatographie pour permettre l'amenée de gaz échantillon et formant un segment de liaison d'une conduite de gaz échantillon, et
le segment de liaison de la conduite de gaz échantillon s'étendant du module d'extraction de gaz (20) à l'injecteur (34) du module de chromatographie (30) et étant exempt de soupape.

2. Dispositif conforme à la revendication 1,
dans lequel le module de chromatographie comporte un bloc de connexion (31) installé sur la plaque de support (33) comprenant des raccordements de gaz porteur et de gaz échantillon (38, 39, 40, 41) permettent de relier les conduites d'entrée de gaz porteur et de gaz échantillon et les conduites de sortie de gaz porteur et de gaz d'échantillon aux capillaires, et le module d'extraction de gaz (20) étant directement connecté en étant hermétiquement amovible au bloc de connexion (31) du module de chromatographie (30).

3. Dispositif conforme à la revendication 1,
dans lequel au moins un dispositif d'extraction de gaz (21, 22) et le segment de liaison (260) de la conduite de gaz échantillon sont installés sur la plaque de support (33) de sorte que le module de chromatographie en phase gazeuse (30) et le dispositif d'extraction de gaz (21, 22) forment un composant intégré du dispositif.

4. Dispositif conforme à l'une des revendications précédentes,
dans lequel le volume mort situé entre le module d'extraction de gaz (20) et le dispositif de chromatographie en phase gazeuse est inférieur à 500 microlitres, de préférence inférieur à 200 microlitres, par exemple inférieur à 100 microlitres et, par exemple situé entre 10 microlitres et 50 microlitres.

5. Dispositif conforme à l'une des revendications précédentes,
dans lequel le temps de réponse est inférieur à 15 mn, de préférence inférieur à 10 mn, par exemple inférieur à 5 mn.

6. Dispositif conforme à l'une des revendications précédentes,
dans lequel la membrane perméable aux gaz (215) du dispositif d'extraction de gaz (21, 22) est au moins une membrane en forme de tube et le dispositif d'extraction de gaz (21, 22) est de préférence un dispositif d'extraction de gaz à double tube ou un dispositif d'extraction de gaz à tubes multiples.

7. Dispositif conforme à l'une des revendications 1 à 5,
dans lequel le dispositif d'extraction de gaz et un micro réacteur à double canal.

8. Dispositif conforme à l'une des revendications précédentes,
comprenant deux soupapes de précision (23, 24) et au l'une des deux soupapes de précision est branchée à l'avant du dispositif d'extraction de gaz (21, 22).

9. Dispositif conforme à la revendication 8,
comprenant une commande des soupapes de précision (23, 24), et l'un des capillaires est un premier capillaire d'entrée de gaz échantillon (40) et l'entrée de gaz échantillon dans l'injecteur (34) est reliée au premier capillaire d'entrée de gaz échantillon, l'entrée de gaz échantillon (40) dans l'injecteur étant commandée par la commande des soupapes de précision de sorte que du gaz échantillon (240) puisse être transféré par le premier capillaire d'entrée de gaz échantillon (40) du module d'extraction (20) vers l'injecteur (34) pour que l'injecteur puisse être alimenté unilatéralement en gaz échantillon par le premier capillaire d'entrée de gaz échantillon (40).

10. Dispositif conforme à la revendication8,
comprenant une commande des soupapes de précision (23, 24) et l'un des capillaires est un premier capillaire d'entrée de gaz échantillon (40) et un autre capillaire est un second capillaire d'entrée de gaz échantillon (41) et l'entrée de gaz échantillon dans l'injecteur (34) est reliée au premier capillaire d'entrée de gaz échantillon (40) et au second capillaire d'entrée de gaz échantillon (41) et l'entrée de gaz échantillon dans l'injecteur est commandée par la commande des soupapes de précision de sorte que du gaz échantillon (240) puisse être transféré par le premier capillaire d'entrée de gaz échantillon et par le second capillaire d'entrée de gaz échantillon (40, 41) du module d'extraction (20) vers l'injecteur (34) pour que l'injecteur puisse être alimenté bilatéralement en gaz échantillon par le premier capillaire d'entrée de gaz échantillon et par le second capillaire d'entrée de gaz échantillon (40, 41).

11. Dispositif conforme à la revendication 10,
comprenant un autre dispositif d'extraction de gaz (21, 22) muni d'une membrane perméable aux gaz, les deux soupapes de précision (23, 24) étant respectivement branchées à l'avant de l'un des deux dispositifs d'extraction de gaz et étant de préférence des soupapes à voies multiples, et la conduite de sortie du gaz échantillon de l'un des dispositifs d'extraction de gaz (21) étant reliée au premier capillaire d'entrée du gaz échantillon (40) et la conduite de sortie du gaz échantillon de l'autre dispositif d'extraction de gaz (22) étant reliée au second capillaire d'entrée de gaz échantillon (41).

12. Dispositif conforme à l'une des revendications précédentes,
dans lequel la membrane (215) renferme un polymère fluoré amorphe, par exemple du polytétrafluoréthylène de préférence du téflon AF 2400 ou du polydiméthylsiloxane (PDMS).

13. Dispositif conforme à l'une des revendications précédentes,
comprenant un chauffage (27) pour permettre de chauffer le liquide avant son entrée dans le module d'extraction (20).

14. Dispositif conforme à l'une des revendications précédentes,
dans lequel le module de chromatographie (30) comporte plus d'une colonne de séparation (35), et au moins l'une des colonne de séparation renferme un garnissage différent des garnissages des autres colonnes de séparation.

15. Dispositif conforme à l'une des revendications précédentes,
comprenant des raccordements d'entrée et de sortie d'un fluide de calibrage pour permettre de calibrer le dispositif.

16. Utilisation d'un dispositif conforme à l'une des revendications précédentes,
pour permettre l'extraction et l'analyse en continu de gaz de transformateur provenant d'une huile de transformateur.
